# EUROPEAN PATENT SPECIFICATION

(11) **EP 2 058 008 B1**
(45) Date of publication and mention of the grant of the patent: **21.08.2013**
(21) Application number: 07790979.4
(22) Date of filing: 19.07.2007
(51) Int. Cl.: A61K 31/197, A61P 3/04, A61P 43/00, A61K 45/00

(54) **ANTI-OBESITY AGENT AND USE THEREOF**
MITTEL GEGEN FETTSUCHT UND SEINE VERWENDUNG
AGENT ANTI-OBÉSITÉ ET SON UTILISATION

(30) Priority: 11.08.2006 JP 2006219246; 05.10.2006 JP 2006273997; 10.04.2007 JP 2007102799
(43) Date of publication of application: 13.05.2009
(73) Proprietor: National University Corporation Nagoya University, Nagoya-shi, Aichi 464-8601 (JP)
(72) Inventor: ARIMA, Hiroshi, Nagoya-shi, Aichi 464-8601 (JP); SATO, Ikuko, Nagoya-shi, Aichi 464-8601 (JP); OISO, Yutaka, Nagoya-shi, Aichi 464-8601 (JP)
(74) Representative: Müller Fottner Steinecke
(86) International application number: PCT/JP2007/064225
(87) International publication number: WO 2008/018275

(56) References cited:
- WO-A1-2005/082372
- JP-A- 06 510 760
- US-A1- 2005 090 554
- ADDAE J I ET AL: "Activation of thermogenesis of brown fat in rats by Baclofen" NEUROPHARMACOLOGY, PERGAMON PRESS, OXFORD, GB, vol. 25, no. 6, 1 June 1986 (1986-06-01), pages 627-631, XP023822795 ISSN: 0028-3908 [retrieved on 1986-06-01]
- ZARRINDAST M R ET AL: "Food intake suppressant effect of baclofen in rats" GENERAL PHARMACOLOGY, PERGAMON PRESS, OXFORD, GB, vol. 20, no. 5, 1 January 1989 (1989-01-01), pages 701-703, XP023819603 ISSN: 0306-3623 [retrieved on 1989-01-01]
- FOLTIN ET AL: "Baclofen decreases feeding in non-human primates" PHARMACOLOGY BIOCHEMISTRY AND BEHAVIOR, ELSEVIER, US, vol. 82, no. 4, 1 December 2005 (2005-12-01), pages 608-614, XP005278534 ISSN: 0091-3057
- BUDA-LEVIN A. ET AL.: 'Baclofen reduces fat intake under binge-type conditions' PHYSIOLOGY & BEHAVIOR vol. 86, no. 1-2, 2005, pages 176 - 184, XP005078013
- SATO IKUKO ET AL.: 'Insulin inhibits neuropeptide Y gene expression in the arcuat nucleus through GABAergic systems' THE JOURNAL OF NEUROSCIENCE vol. 25, no. 38, 2005, pages 8657 - 8664, XP003020975

## Description

### Technical Field

The present invention relates to an anti-obesity medicament and a method of treating obesity, and the like.

### Background Art

It has been revealed that leptin deficiency is a cause of obesity and diabetes by identifying a pathogenic gene of an ob/ob mouse that is a model animal for obesity and diabetes in 1994 (Non-patent Document 1). Leptin, which is secreted by fat cells, acts on the hypothalamic arcuate nucleus, that is the primary site for food intake regulation, to suppress expression of neuropeptide Y (NPY) that is an orexigenic peptide synthesized in the arcuate nucleus, and enhances expression of proopiomelanocortin (POMC) that is a precursor of α-MSH, an anorexigenic peptide, to thereby control an energy balance toward the negative direction (Non-patent Documents 2 and 3). However, in general, an obese person has a high blood leptin concentration and therefore a sufficient anti-obesity action is not exerted by administration of leptin to the obese person (Non-patent Documents 4 and 5). Such a result suggests a possibility that leptin resistance, that is, a leptin signal disorder is a cause of obesity development.

Main anti-obesity medicaments clinically applied at present include Mazindol that is an inhibitor of intracerebral noradrenaline reuptake, Sibutramine that is an inhibitor of intracerebral noradrenaline and serotonin reuptake, Rimonabant that is a selective antagonist of a Cannabinoid-1 receptor, and the like; however, none of the medicaments has an action of suppressing NPY or enhancing POMC. On the other hand, it is suggested that the ciliary neurotrophic factor (CNTF) that is a neurotrophic factor shows an anti-obesity action by the same signaling system as in leptin and clinical application thereof has been promoted currently, but there is a problem such that an antibody to CNTF is generated in a patient to which CNTF is administered (Non-patent Document 6).
Non-patent Document 1: Zhang. Y. et al. Nature. 1994. 372: 425-432.
Non-patent Document 2: Stephens, T. W. et al. Nature. 1995. 377: 530-532.
Non-patent Document 3: Schwartz, M. W. et al. Diabetes. 1997. 46: 2119-2123.
Non-patent Document 4: Considine, R. V. et al. The New England Journal of Medicine. 1996. 334: 292-295.
Non-patent Document 5: Heymsfield, S. B. et al. JAMA. 1999. 282: 1568-1575.
Non-patent Document 6: Korner, J. et al. The Journal of Clinical Endocrinology & Metabolism. 2004. 89: 2616-2621.
Non-patent Document 7: Sato, I. et al. The Journal of Neuroscience. 2005. 25: 8657-8664.
Non-patent Document 8: Niswender, K. D. et al. Frontiers in Neuroendocrinology. 2003. 24: 1-10.

Addae et al., Neuropharmacology 25 (1986), 627-631 describe activation of thermogenesis of brown fat in rats by baclofen.

Zarrindast et al., Gen. Pharmac. 20 (1989), 701-703 describe the food intake suppressant effect of baclofen in rats under predetermined experimental conditions.

Foltin, Pharmacology, Biochemistry and Behavior 82 (2005), 608-614 reviews the literature concerning the reported effects of baclofen on eating behavior and reports on a study which findings suggest that the effects of baclofen in non-human primates are due to non-specific behavioral disruptions.

US 2005/0090554 A1 describes the use of baclofen for the treatment of gastroparesis and nonulcer dyspepsia.

Buda-Levin et al., Physiol. Behav. 15 (2005), 176-184 describe experiments with rats in which baclofen reduces food intake under binge-type conditions.

WO 93/03714 relates to a method for the treatment of non-insulin (Type II) diabetes mellitus. While some compounds are claimed to be useful in the treatment of excess adiposity or obesity, the experiments described in this application are not suitable to conclude the usefulness of for example baclofen at a therapeutic level.

### Disclosure of the Invention

### Problem to be Solved by the Invention

It is desired to discover a medicament exerting an anti-obesity effect by acting on NPY neurons and POMC neurons in the arcuate nucleus in place of leptin. An object of the present invention is to meet such a desire, that is, to provide a novel anti-obesity medicament and a treating method using the medicament.

### Means for Solving the Problem

The inventors of the present invention have clarified in a study using hypothalamic organ culture that insulin that is a food intake suppressing hormone suppresses NPY expression in the arcuate nucleus via GABA neurons (Non-patent Document 7). After this finding was obtained, the present inventors focused attentions on recognition of cross talk in the signaling system of insulin and leptin in the hypothalamus (Non-patent Document 8), and have reached an idea that there is a possibility that activation of the GABA system in the hypothalamic arcuate nucleus enhances the signal downstream leptin receptor. Enhancement of the leptin signal leads to exertion of an anti-obesity effect. Based on such an idea, the present inventors intensively studied in order to find a medicament exerting an anti-obesity effect by acting on NPY neurons and POMC neurons. First, they focused on a GABA_{B} receptor and made a hypothesis that a GABA_{B} receptor agonist might exert an anti-obesity effect through enhancement of the leptin signal. Then, they established an experimental system in which baclofen as a typical example of GABA_{B} receptor agonists was administered to an obese model animal (db/db mouse of leptin receptor deficient model) and tried to verify this hypothesis. As a result, in the baclofen treated group, significant food intake suppression and body weight increase suppression were observed and significant increases in a blood leptin concentration and a blood adiponectin concentration were also recognized (see Examples described later for the details). Further, in the hypothalamic arcuate nucleus of the baclofen treated group, suppression of NPY mRNA expression and enhancement of POMC mRNA expression were observed, which suggested that baclofen exerted an anti-obesity effect through the action on NPY neurons and POMC neurons in the arcuate nucleus. While this result supports effectiveness of baclofen as an anti-obesity medicament, the result also indicates that, in addition to baclofen, substances capable of enhancing signaling via a GABA_{B} receptor in the same manner as baclofen, that is, other GABA_{B} receptor agonists are also prospective candidates for an anti-obesity medicament.
Further, also in a diet-induced obese mouse (DIO mouse) by administration of high fat-diet, that is an obese model animal close to human obesity disease, administration of baclofen showed an effect of food intake suppression and an effect of weight increase suppression, and suppressed expression of NPY mRNA and enhanced expression of POMC mRNA in the hypothalamic arcuate nucleus. Further, when 3-aminopropyl(methyl)phosphinic acid and
3-aminopropylphosphonic acid, both of which are other GABA_{B} receptor agonists, were administered to DIO mice, an anti-obesity effect similar to that of baclofen was shown.
By the way, baclofen exists in the (R) form (R-enantiomer) and the (S) form (S-enantiomer), and as a result of further study, a particularly strong anti-obesity effect was re observed in the former.
The present invention is mainly based on the above described findings, and a first aspect of the present invention provides an anti-obesity medicament in the following.
[1] An anti-obesity medicament containing a GABA_{B} receptor agonist or a pharmacologically acceptable salt thereof as an active ingredient.
[2] The anti-obesity medicament according to [1], wherein the GABA_{B} receptor agonist is baclofen.
[3] The anti-obesity medicament according to [2], wherein the baclofen is in the (R) form.
[4] The anti-obesity medicament according to any one of [1] to [3], wherein the medicament acts on NPY neurons and POMC neurons in the hypothalamic arcuate nucleus and thereby exerts an effect of anti-obesity.
[5] The anti-obesity medicament according to any one of [1] to [3], wherein the medicament exerts an effect of anti-obesity on obesity due to leptin resistance or obesity accompanied by leptin resistance.

The present invention also relates to use of a GABA_{B} receptor agonist or a pharmacologically acceptable salt thereof shown below.
[6] Use of a GABA_{B} receptor agonist or a pharmacologically acceptable salt thereof for manufacturing an anti-obesity medicament.
[7] The use according to [6], wherein the GABA_{B} receptor agonist is baclofen.
[8] The use according to [7], wherein the baclofen is in the (R) form.

The present invention further relates to a method of treating obesity shown below.
[9] A method of treating obesity including administering to a subject in need of therapy for obesity a therapeutically effective amount of a GABA_{B} receptor agonist or a pharmacologically acceptable salt thereof.
[10] The method of treating obesity according to [9], wherein the GABA_{B} receptor agonist is baclofen.
[11] The method of treating obesity according to [10], wherein the baclofen is in the (R) form.

### Brief Description of Drawings

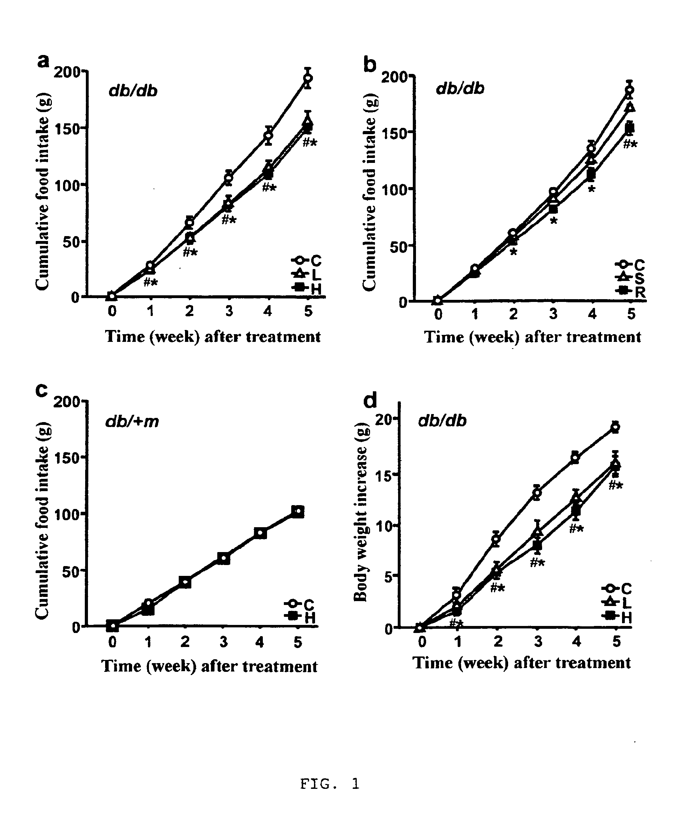

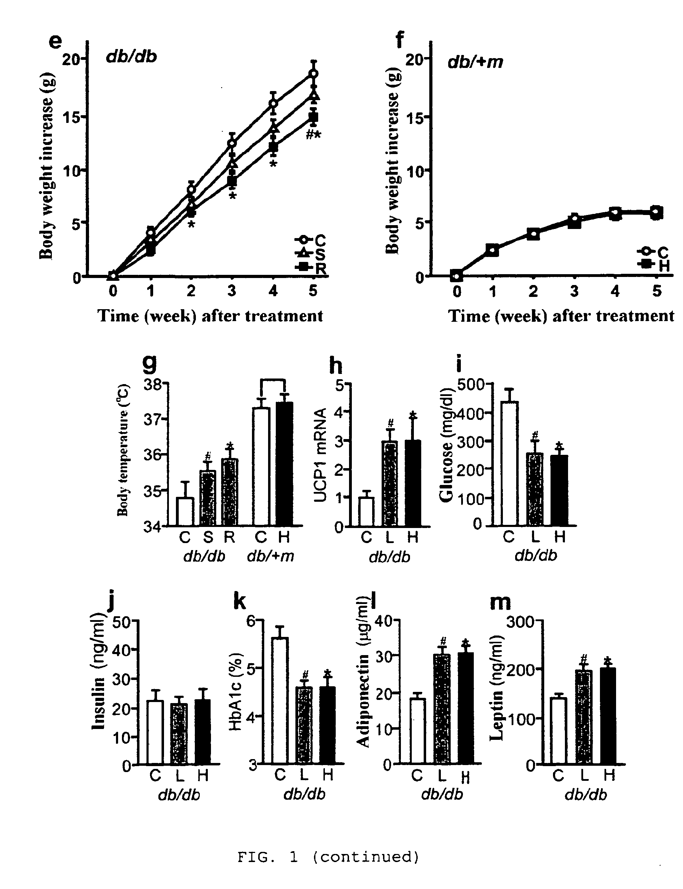

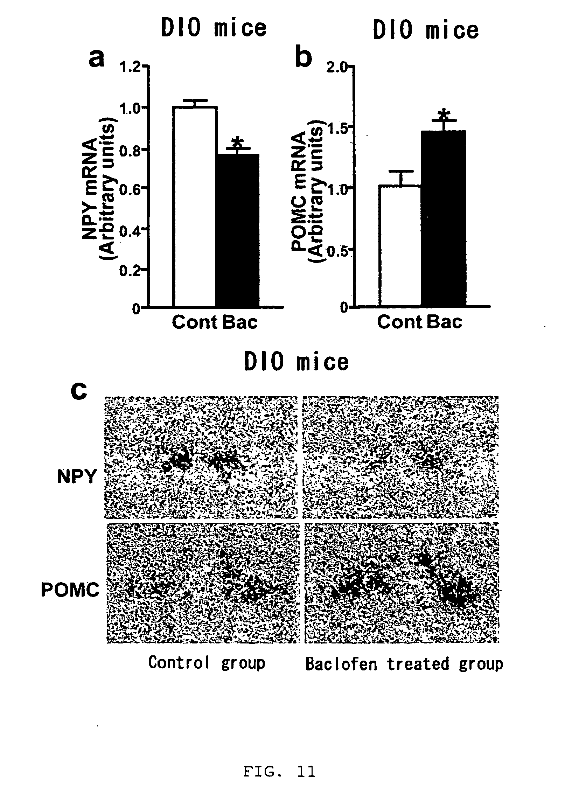

Figs. 1a to 1m show graphs illustrating actions of continuous baclofen administration on db/db mice and db/⁺m mice (C: control group, L: low concentration baclofen racemic mixture treated group, H: high concentration baclofen racemic mixture treated group, S: S-baclofen treated group, R: R-baclofen treated group, db/db: db/db mice, db/⁺m: db/⁺m mice. #: P < 0.05 control to low concentration baclofen racemic mixture treated group, or control to S-baclofen treated group. *: P < 0.05 control to high concentration baclofen racemic mixture treated group, or control to R-baclofen treated group). Figs. 1a and 1d show graphs illustrating actions of a baclofen racemic mixture on db/db mice (a: cumulative food intake amount, d: body weight increase). Continuous administration of the baclofen racemic mixture for 5 weeks significantly suppressed cumulative food intake amounts (a) and body weight increases (d) from 1 week after the start of the administration, and the significant suppression continued until the end of the experiment. Figs. 1b and 1e show graphs illustrating actions of R-baclofen and S-baclofen on db/db mice (b: cumulative food intake amount, e: body weight increase). Continuous administration of R-baclofen for 5 weeks significantly suppressed cumulative food intake amounts (b) and body weight increases (e) from 2 weeks after the start of the administration, and the significant suppression continued until the end of the experiment. On the other hand, in continuous administration of S-baclofen for 5 weeks, significant effects were not given on the cumulative food intake amounts (b) and the body weight increases (e) for 4 weeks from the start of the administration, but the cumulative food intake amounts (b) and the body weight increases (e) were significantly suppressed 5 weeks after the start of the administration. Figs. 1c and 1f show graphs illustrating actions of a baclofen racemic mixture on db/⁺m mice (c: cumulative food intake amount, f: body weight increase). Continuous administration of the baclofen racemic mixture for 5 weeks did not give significant effects on cumulative food intake amounts (c) and body weight increases (f) during the administration period. Fig. 1g illustrates comparison of changes in body temperatures due to baclofen administration between db/db mice and db/⁺m mice. Baclofen administration significantly increased body temperatures in db/db mice, but did not give a significant effect on body temperatures of db/⁺m mice. Fig. 1h illustrates comparison of UCP1 mRNA expression amounts in brown adipose tissues in db/db mice. Baclofen administration significantly enhanced UCP1 mRNA expression. Fig. 1i illustrates comparison of fasting blood glucose levels in db/db mice. Baclofen administration significantly decreased the fasting blood glucose levels. Fig. 1j illustrates comparison of fasting blood insulin concentrations in db/db mice. Baclofen administration did not give a significant effect on the fasting blood insulin concentrations. Fig. 1k illustrates comparison of HbA1c values in db/db mice. Baclofen administration significantly decreased the HbA1c values. Fig. 11 illustrates comparison of blood adiponectin concentrations in db/db mice. Baclofen administration significantly increased the blood adiponectin concentrations. Fig. 1m illustrates comparison of blood leptin concentrations in db/db mice. Baclofen administration significantly increased the blood leptin concentrations.

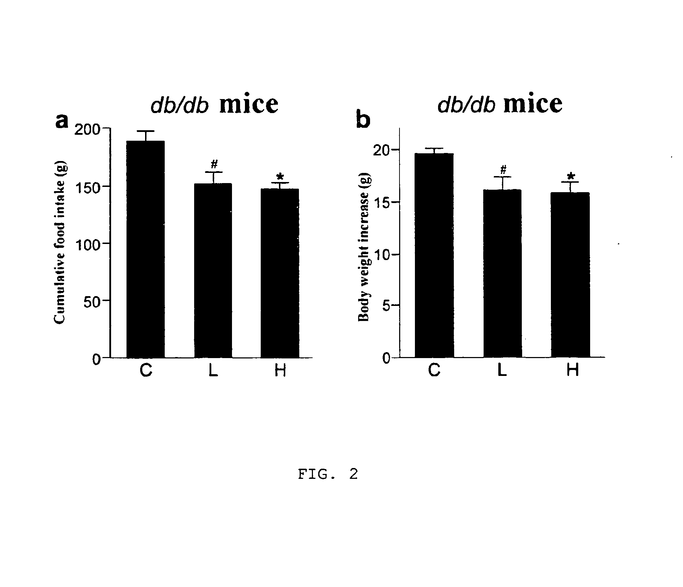

Figs. 2a and 2b show graphs illustrating cumulative food intake amounts and body weight increases in db/db mice after 5-week baclofen administration (C: control group, L: low concentration baclofen racemic mixture treated group, H: high concentration baclofen racemic mixture treated group. #: P < 0.05 control to low concentration baclofen racemic mixture treated group. *: P < 0.05 control to high concentration baclofen racemic mixture treated group).

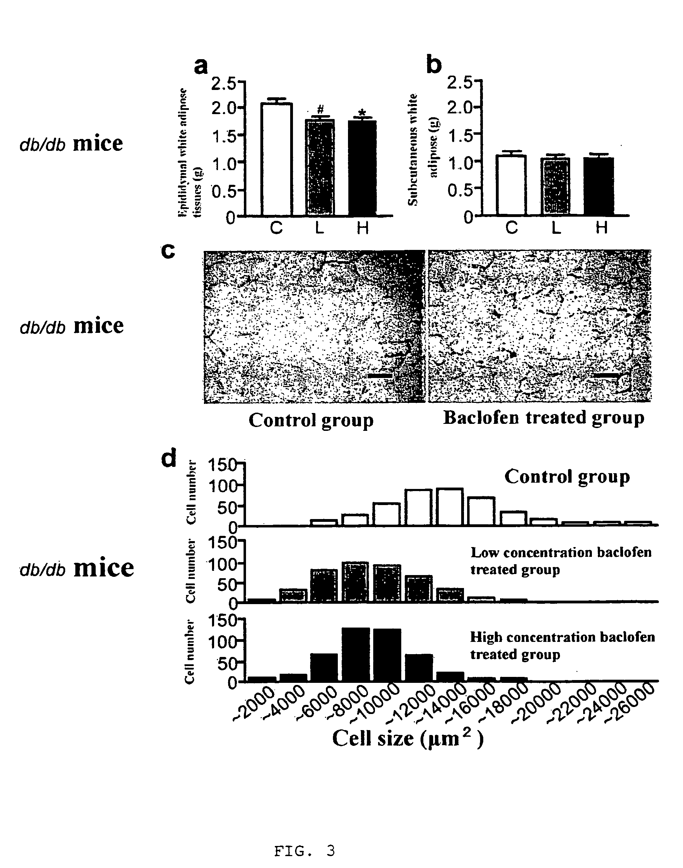

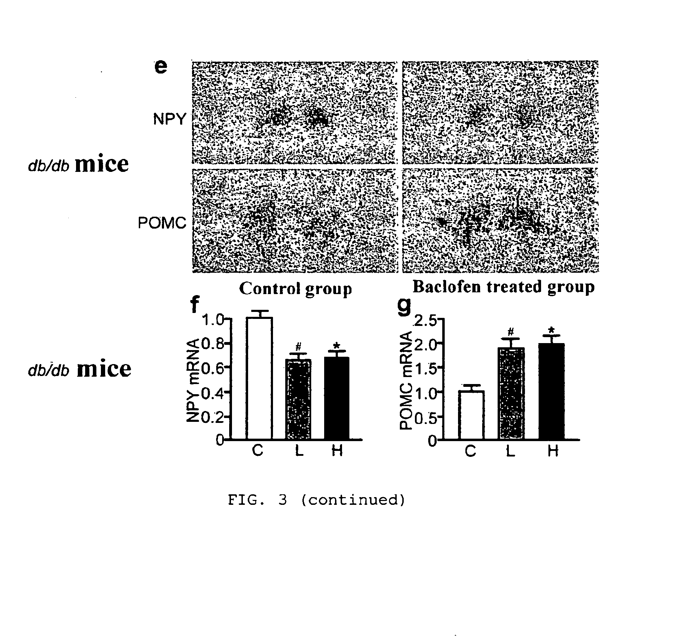

Figs. 3a, 3b, 3d, 3f and 3g show graphs illustrating effects of continuous baclofen administration to db/db mice on adipose tissues and NPY and POMC expression in the hypothalamic arcuate nuclei (C: control group, L: low concentration baclofen racemic mixture treated group, H: high concentration baclofen racemic mixture treated group. #: P < 0.05 control to low concentration baclofen racemic mixture treated group. *: P < 0.05 control to high concentration baclofen racemic mixture treated group). Fig. 3a illustrates comparison of weights of epididymal white adipose tissues. The baclofen administration significantly decreased weights of epididymal white adipose tissues. Fig. 3b illustrates comparison of weights of subcutaneous white adipose tissues. The baclofen administration did not give a significant effect on weights of the subcutaneous white adipose tissues. Fig. 3c shows H-E stain images of epididymal white adipose tissues (the scale bar indicates 100 µm). As compared with the control group (left), fat cells are recognized to become small in the baclofen treated group (right). Fig. 3d illustrates comparison of adipose sizes in the H-E stain images (from the upper graph, sequentially showing the control group, the low concentration baclofen treated group and the high concentration baclofen treated group). As compared with the control group, a decrease in sizes of fat cells is recognized in the low concentration baclofen treated group and the high concentration baclofen treated group. Fig. 3e shows photos of typical in situ hybridization. The upper left photo shows expression of NPY mRNA in the control group. The upper right photo shows expression of NPY mRNA in the baclofen treated group. The bottom left photo shows expression of POMC mRNA in the control group. The bottom right photo shows expression of POMC mRNA in the baclofen treated group. Fig. 3f illustrates comparison of NPY mRNA expression amounts. Significant suppression of NPY mRNA expression was recognized in the baclofen treated groups. Fig. 3g illustrates comparison of POMC mRNA expression amounts. Significant increases in POMC mRNA expression were recognized in the baclofen treated groups.

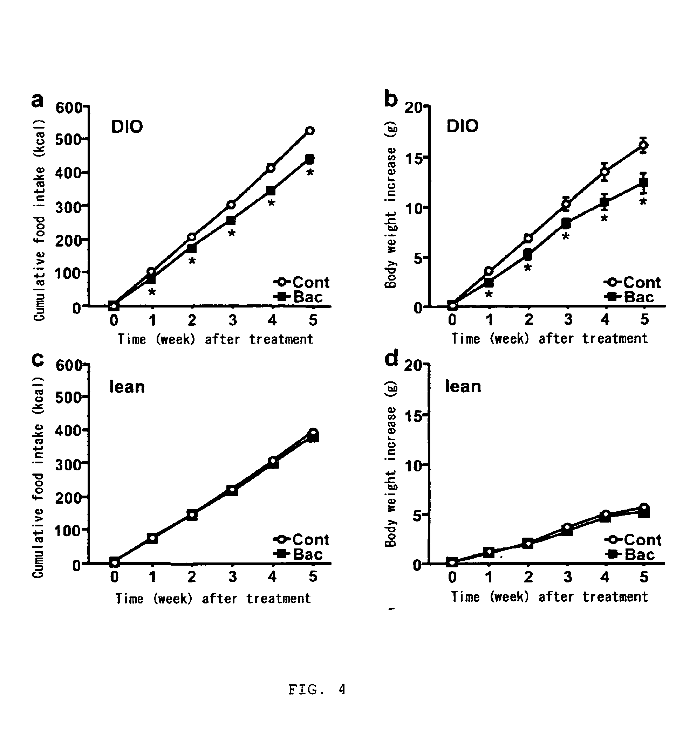

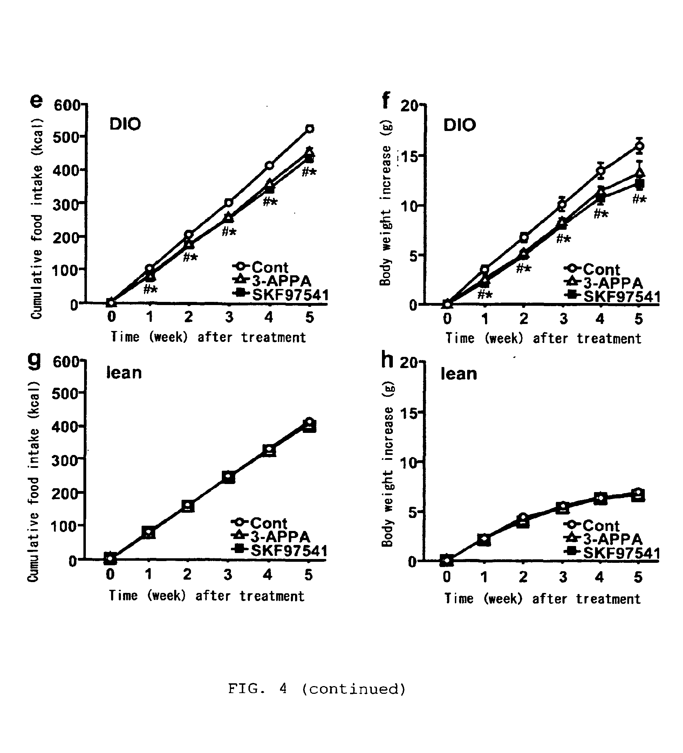

Figs. 4a to 4h show graphs illustrating actions of continuous administration of a baclofen racemic mixture, 3-aminopropyl-(methyl) phosphinic acid (SKF97541) or 3-aminopropylphosphonic acid (3-APPA), any of which is a GABA_{B} receptor agonist, on high fat diet administered obese mice (DIO mice) and normal diet administered non-obese mice (lean mice) (Cont: control group, Bac: baclofen racemic mixture treated group, 3-APPA: 3-APPA treated group, SKF97541: SKF97541treated group, DIO: DIO mice, lean: lean mice, *: P < 0.05 control group to baclofen racemic mixture treated group, or control group to SKF97541 treated group, #: P < 0.05 control group to 3-APPA treated group). Figs. 4a and 4b show graphs illustrating actions of a baclofen racemic mixture on DIO mice (a: cumulative food intake amount, b: body weight increase). The continuous administration of a baclofen racemic mixture for 5 weeks significantly suppressed cumulative food intake amounts (a) and body weight increases (b) from 1 week after the start of the administration, and the significant suppression continued until the end of the experiment. Figs. 4c and 4d show graphs illustrating actions of a baclofen racemic mixture on lean mice (c: cumulative food intake amount, d: body weight increase). Continuous administration of the baclofen racemic mixture for 5 weeks did not give significant effects on cumulative food intake amounts (c) and body weight increases (d) of the lean mice in the administration period. Figs. 4e and 4f show graphs illustrating actions of SKF97541 and 3-APPA on DIO mice (e: cumulative food intake amount, f: body weight increase). Continuous administration of SKF97541 and 3-APPA for 5 weeks significantly suppressed cumulative food intake amounts (e) and body weight increases (f) from 1 week after the start of the administration, and the significant suppression continued until the end of the experiment.
Figs. 4g and 4h show graphs illustrating actions of SKF97541 and 3-APPA on lean mice (g: cumulative food intake amount, h: body weight increase). Continuous administration of SKF97541 and 3-APPA for 5 weeks did not give significant effects on cumulative food intake amounts (g) and body weight increases (h) of the lean mice in the administration period.

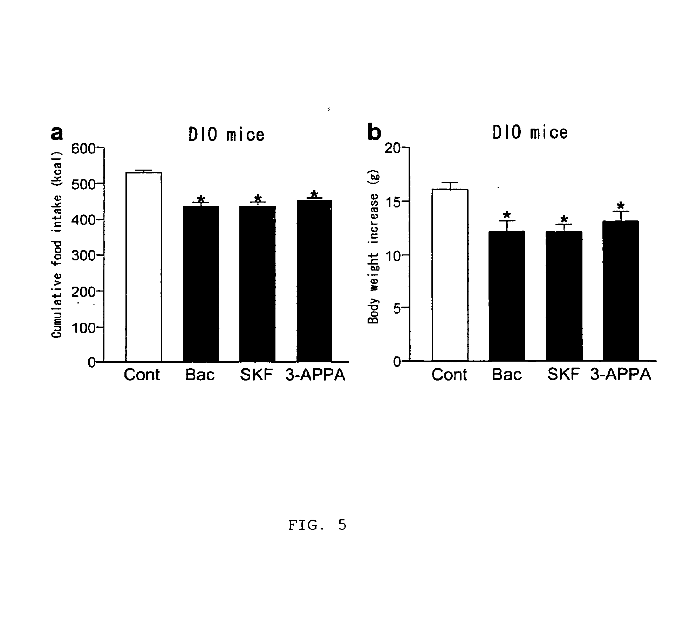

Figs. 5a and 5b show graphs illustrating cumulative food intake amounts and body weight increases in DIO mice after 5-week continuous administration of a baclofen racemic mixture, SKF97541 or 3-APPA, any of which is a GABA_{B} receptor agonist (Cont: control group, Bac: baclofen racemic mixture treated group, SKF: SKF97541 treated group, 3-APPA: 3-APPA treated group. *: P < 0.05 control group to baclofen racemic mixture treated group, or SKF97541 treated group, or 3-APPA treated group). Fig. 5a illustrates comparison of cumulative food intake amounts in 5 weeks in DIO mice after 5-week continuous administration of the GABA_{B} receptor agonists (a baclofen racemic mixture, SKF97541 or 3-APPA). Significant decreases in cumulative food intake amounts were recognized in all of the baclofen racemic mixture treated group, the SKF97541 treated group and the 3-APPA treated group as compared with the control group. Fig. 5b illustrates comparison of body weight increases in 5 weeks in DIO mice after 5-week continuous administration of GABA_{B} receptor agonists (a baclofen racemic mixture, SKF97541 or 3-APPA). Significant reduction of body weight increases was recognized in all of the baclofen racemic mixture treated group, the SKF97541 treated group and the 3-APPA treated group as compared with the control group.

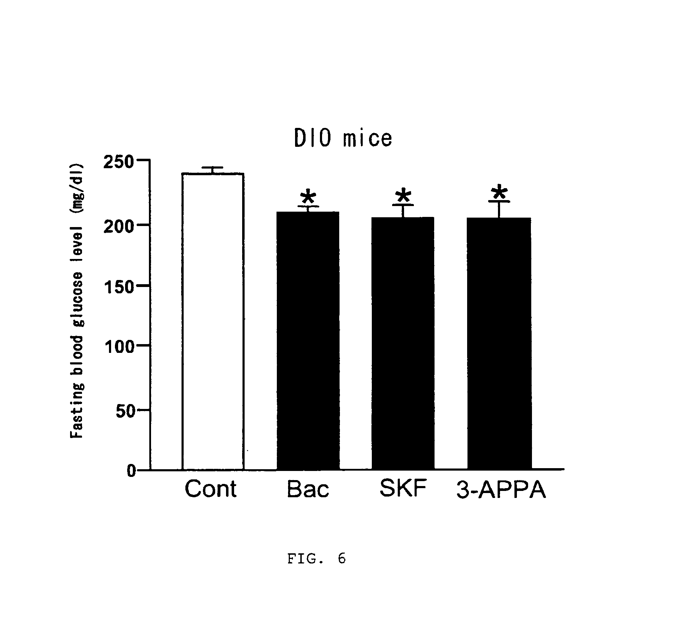

Fig. 6 shows a graph illustrating fasting blood glucose levels after fasting for 6 hours in DIO mice after 5-week continuous administration of a baclofen racemic mixture, SKF97541 or 3-APPA, any of which is a GABA_{B} receptor agonist (Cont: control group, Bac: baclofen racemic mixture treated group, SKF: SKF97541 treated group, 3-APPA: 3-APPA treated group. *: P < 0.05 control group to baclofen racemic mixture treated group, or SKF97541 treated group, or 3-APPA treated group). Significant decreases in fasting blood glucose levels were recognized in all of the baclofen racemic mixture treated group, the SKF97541 treated group and the 3-APPA treated group as compared with the control group.

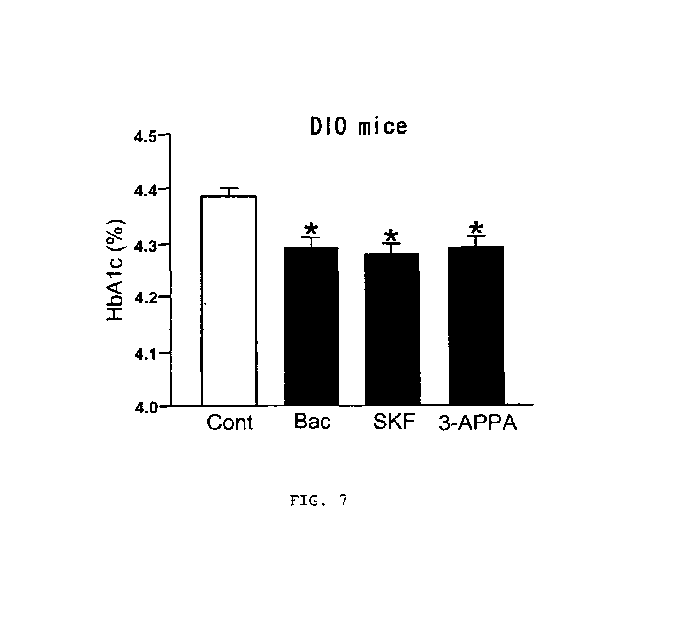

Fig. 7 shows a graph illustrating HbA1c values in DIO mice after 5-week continuous administration of a baclofen racemic mixture, SKF97541 or 3-APPA, any of which is a GABA_{B} receptor agonist (Cont: control group, Bac: baclofen racemic mixture treated group, SKF: SKF97541 treated group, 3-APPA: 3-APPA treated group. *: P < 0.05 control group to baclofen racemic mixture treated group, or SKF97541 treated group, or 3-APPA treated group). Significant decreases in the HbA1c values were recognized in all of the baclofen racemic mixture treated group, the SKF97541 treated group and the 3-APPA treated group as compared with the control group.

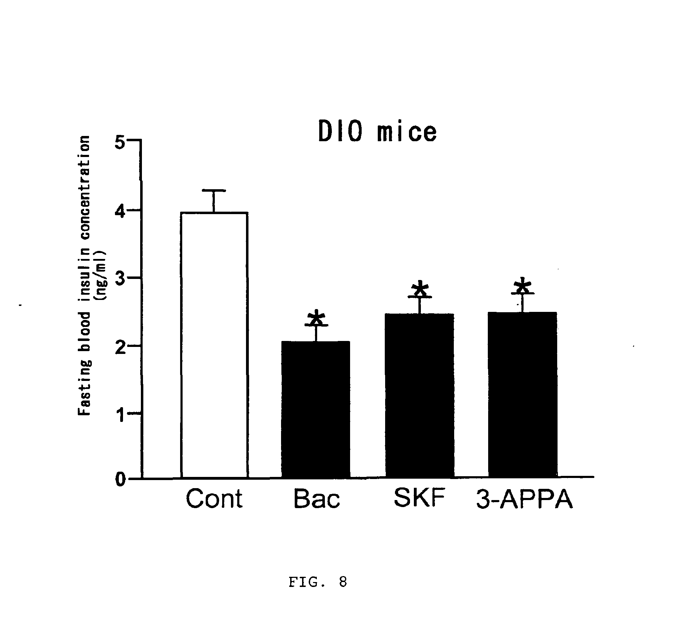

Fig. 8 shows a graph illustrating fasting blood insulin concentrations after fasting for 6 hours in DIO mice after 5-week continuous administration of a baclofen racemic mixture, SKF97541 or 3-APPA, any of which is a GABA_{B} receptor agonist (Cont: control group, Bac: baclofen racemic mixture treated group, SKF: SKF97541 treated group, 3-APPA: 3-APPA treated group. *: P < 0.05 control group to baclofen racemic mixture treated group, or SKF97541 treated group, or 3-APPA treated group). Significant decreases in fasting blood insulin concentrations were recognized in all of the baclofen racemic mixture treated group, the SKF97541 treated group and the 3-APPA treated group as compared with the control group.

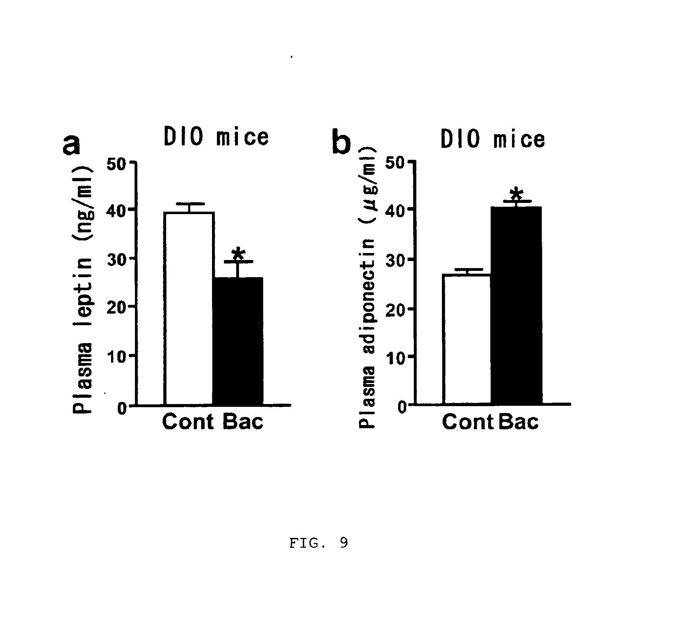

Figs. 9a and 9b show graphs illustrating changes in plasma leptin concentrations and plasma adiponectin concentrations in DIO mice after 5 week-continuous administration of a baclofen racemic mixture (Cont: control group, Bac: baclofen racemic mixture treated group. *: P < 0.05 control group to baclofen racemic mixture treated group). Fig. 9a shows that administration of the baclofen racemic mixture significantly decreased the plasma leptin concentrations in DIO mice. Fig. 9b shows that administration of the baclofen racemic mixture significantly increased the plasma adiponectin concentrations in DIO mice.

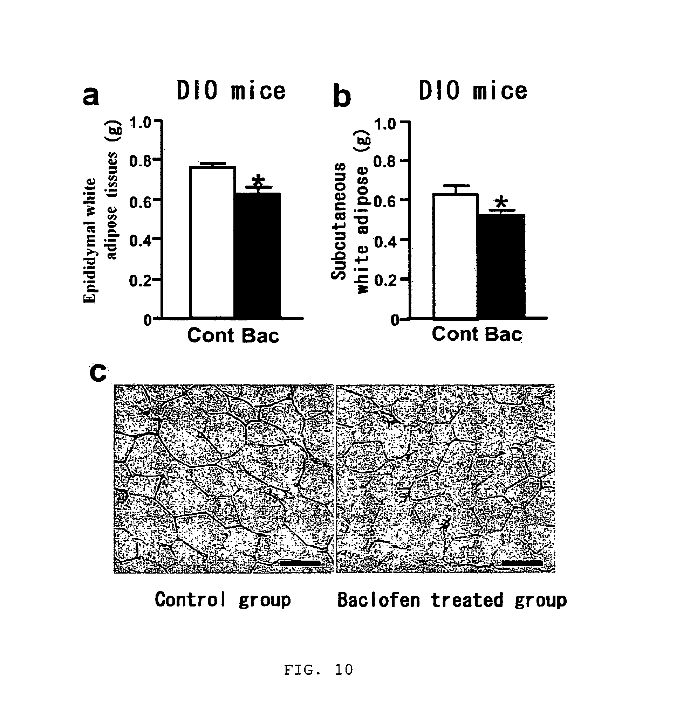

Figs. 10a and 10b show graphs illustrating effects of 5-week continuous administration of a baclofen racemic mixture on white adipose tissues in DIO mice (Cont: control group, Bac: baclofen racemic mixture treated group. *: P < 0.05 control group to baclofen racemic mixture treated group). Fig. 10a shows that administration of the baclofen racemic mixture significantly decreased weights of epididymal white adipose tissues in DIO mice. Fig. 10b shows that administration of the baclofen racemic mixture significantly decreased weights of subcutaneous white adipose tissues in DIO mice. Fig. 10c shows H-E stain images of the epididymal white adipose tissues (the scale bar indicates 100 µm). As compared with the control group (left), fat cells are recognized to become small in the baclofen treated group (right).
Figs. 11a and 11b show graphs illustrating effects of 5-week continuous administration of a baclofen racemic mixture on NPY mRNA and POMC mRNA expression amounts in the hypothalamic arcuate nuclei in DIO mice (Cont: control group, Bac: baclofen racemic mixture treated group. *: P < 0.05 control group to baclofen racemic mixture treated group). Fig. 11a illustrates comparison of NPY mRNA expression amounts. Significant suppression of the NPY mRNA expression amounts was recognized in the baclofen racemic mixture treated group. Fig. 11b illustrates comparison of POMC mRNA expression amounts. A significant increase in the POMC mRNA expression amount was recognized in the baclofen racemic mixture treated group. Fig. 11c shows photos of typical in situ hybridization. The upper left photo shows expression of NPY mRNA in the control group. The upper right photo shows expression of NPY mRNA in the baclofen racemic mixture treated group. The bottom left photo shows expression of POMC mRNA in the control group. The bottom right photo shows expression of POMC mRNA in the baclofen racemic mixture treated group.

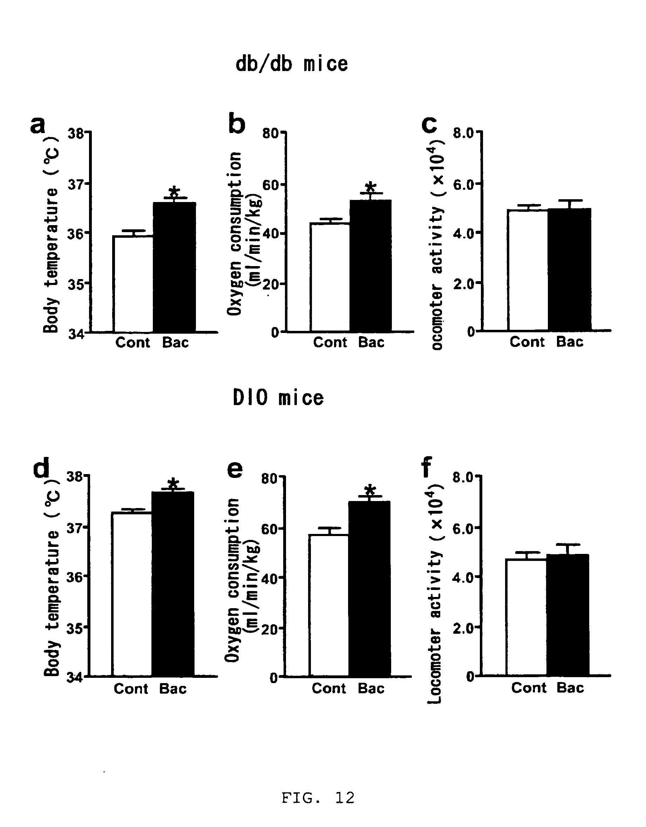

Figs. 12a to 12f show graphs illustrating effects of 5-week continuous administration of a baclofen racemic mixture on heat production and motor activity in DIO mice and db/db mice (Cont: control group, Bac: baclofen racemic mixture treated group. *: P < 0.05 control group to baclofen racemic mixture treated group). Figs. 12a and 12d illustrate comparison of changes in body temperatures due to baclofen administration. Administration of the baclofen racemic mixture significantly increased body temperatures in db/db mice (a) and DIO mice (d). Figs. 12b and 12e illustrate comparison of changes in oxygen consumptions due to baclofen administration. The administration of the baclofen racemic mixture significantly increased oxygen consumptions in db/db mice (b) and DIO mice (e). Figs. 12c and 12f illustrate comparison of changes in motor activity due to baclofen administration. The administration of the baclofen racemic mixture did not give a significant effect on motor activity in db/db mice (c) and DIO mice (f).

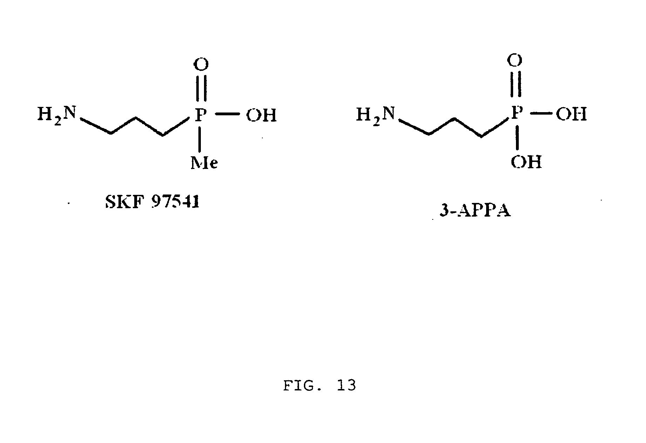

Fig. 13 shows structural formulae of SKF97541 and 3-APPA.

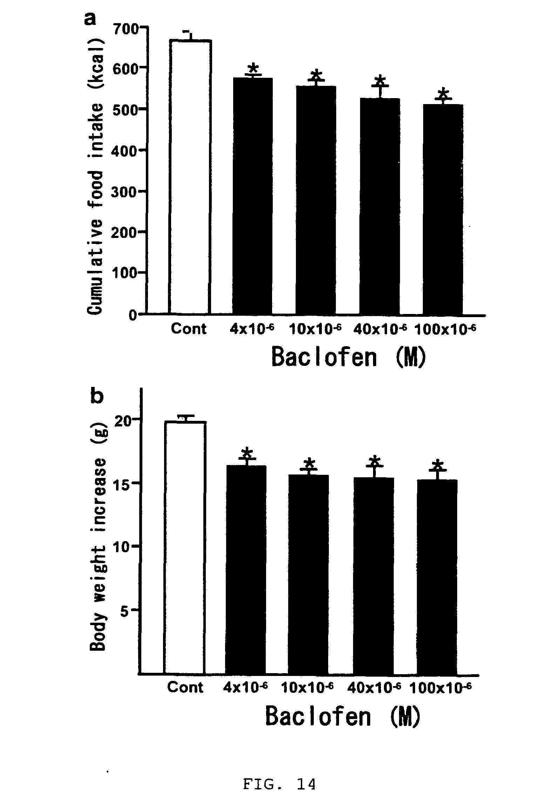

Figs. 14a and 14b show graphs illustrating effects of administration of a baclofen racemic mixture on cumulative food intake amounts (a) and body weight increases (b) in 5 weeks in db/db mice (Cont: control group, 4 × 10⁻⁶ M, 10 × 10⁻⁶ M, 40 × 10⁻⁶ M and 100 × 10⁻⁶ M indicate concentrations of baclofen dissolved in drinking water in each baclofen treated group. *: P < 0.05 control group to each baclofen racemic mixture treated group). Continuous administration of the baclofen racemic mixture for 5 weeks to db/db mice significantly suppressed the cumulative food intake amounts (a) and the body weight increases (b) in all concentrations from 4 × 10⁻⁶ M to 100 × 10⁻⁶ M.

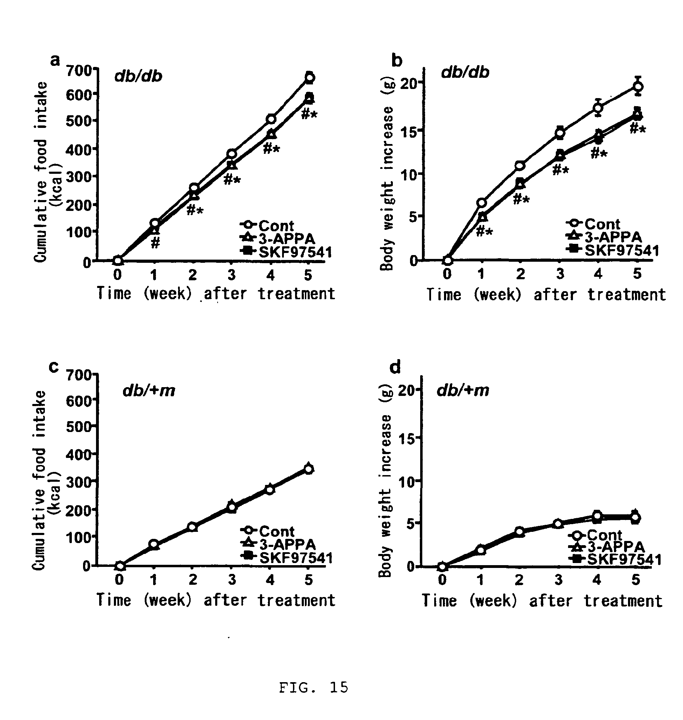

Figs. 15a to 15d show graphs illustrating actions of continuous administration of 3-aminopropyl-(methyl) phosphinic acid (SKF97541) or 3-aminopropylphosphonic acid (3-APPA) on db/db mice and db/⁺m mice (Cont: control group, 3-APPA: 3-APPA treated group, SKF97541: SKF97541 treated group, db/db: db/db mice, db/⁺m: db/⁺m mice. *: P < 0.05 control group to SKF97541 treated group. #: P < 0.05 control group to 3-APPA treated group). Figs. 15a and 15b show graphs illustrating actions of SKF97541 and 3-APPA on db/db mice (a: cumulative food intake amount, b: body weight increase). The cumulative food intake amounts (a) were significantly suppressed from 1 week after the start of the administration of 3-APPA and 2 weeks after the start of the administration of SKF97541, and the significant suppression continued until the end of the experiment. Further, administration of SKF97541 and 3-APPA significantly suppressed body weight increases (b) from 1 week after the start of the administration, and the significant suppression continued until the end of the experiment. Figs. 15c and 15d show graphs illustrating actions of SKF97541 and 3-APPA on db/⁺m mice (c: cumulative food intake amount, d: body weight increase). The continuous administration of SKF97541 and 3-APPA for 5 weeks did not give significant effects on cumulative food intake amounts (c) and body weight increases (d) of the db/⁺m mice in the administration period.

### Best Mode for Carrying Out the Invention

A first aspect of the present invention relates to an anti-obesity medicament. The anti-obesity medicament of the present invention (hereinafter also referred to as "the medicament of the present invention") contains a GABA_{B} receptor agonist or a pharmacologically acceptable salt thereof as an active ingredient. That is, an agonist for a GABA_{B} receptor is used in the present invention. Examples of the GABA_{B} receptor agonist include baclofen, 3-aminopropyl(methyl)phosphinic acid (SKF97541), 4-amino-3-(5-chloro-2-thienyl)-butanoic acid, and 3-aminopropylphosphonic acid. One preferable example of the GABA_{B} receptor agonist is baclofen. That is, in one preferable embodiment of the present invention, an anti-obesity medicament containing baclofen or a pharmacologically acceptable salt thereof as an active ingredient is provided. Baclofen (chemical name: (RS)-4-amino-3-(4-chlorophenyl)butanoic acid, molecular formula: C₁₀H₁₂ClNO₂, molecular weight: 213.66) is expressed by the following chemical formula: 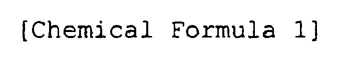 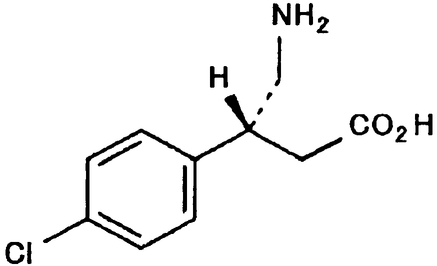 Baclofen exists in the (R) form (R-enantiomer) and in the (S) form (S-enantiomer). In the present invention, either of these enantiomers or a mixture of the both forms (for example, a racemic body) is used. As shown in Examples described later, as a result of a study by the present inventors, it was revealed that the (R) form exerted a higher anti-obesity effect than the (S) form does. Based on this finding, (R) form baclofen is used in a preferable embodiment of the present invention. In addition, baclofen can be prepared, for example, by a method described in US Patent No. 3471548. As a medicament containing baclofen as an active ingredient, Lioresal (registered trademark, Novartis Pharma K.K.) and Gabalon (registered trademark, Daiichi Pharmaceutical Co., Ltd.) are commercially available. For example, R(+)-Baclofen hydrochloride (catalog No. G013, CAS Number 63701-55-3, Sigma Co.) is commercially available as (R) form baclofen, and S (-) -Baclofen hydrochloride (catalog No. G014, CAS Number 63701-56-4, Sigma Co.) is commercially available as (S) form baclofen.

As an active ingredient of the medicament of the present invention, a pharmacologically acceptable salt of a GABA_{B} receptor agonist may be used. The "pharmacologically acceptable salt" means, for example, an acid addition salt, a metal salt, an ammonium salt, an organic amine addition salt, or an amino acid addition salt. Examples of the acid addition salt include inorganic acid salts such as hydrochloride, sulfate, nitrate, phosphate and hydrobromide, and organic acid salts such as acetate, maleate, fumarate, citrate, benzenesulfonate, benzoate, malate, oxalate, methanesulfonate and tartrate. Examples of the metal salt include alkali metal salts such as a sodium salt, a potassium salt and a lithium salt, alkaline earth metal salts such as a magnesium salt and a calcium salt, an aluminum salt and a zinc salt. Examples of the ammonium salt include salts of ammonium and tetramethylammonium. Examples of the organic amine addition salt include a morpholine addition salt and a piperidine addition salt. Examples of the amino acid addition salt include a glycine addition salt, a phenylalanine addition salt, a lysine addition salt, an asparaginic acid addition salt and a glutamic acid addition salt.

The medicament of the present invention is used for treatment or prevention of obesity. The present inventors have revealed from experiments using animal models that a GABA_{B} receptor agonist has both of an action of suppressing expression of NPY mRNA and an action of enhancing expression of POMC mRNA, as shown in Examples described later. As led out from this finding, the medicament of the present invention containing a GABA_{B} receptor agonist (or a pharmacologically acceptable salt thereof) as an active ingredient exerts an anti-obesity effect by acting on NPY neurons and POMC neurons in the hypothalamic arcuate nucleus. With the medicament of the present invention, in place of leptin, an effect of enhancing signaling via NPY neurons and POMC neurons is exerted. Accordingly, the medicament of the present invention can be recognized as being particularly effective for obesity due to leptin resistance or obesity accompanied by leptin resistance.
Herein, "obesity" generally means a state of excessively accumulating adipose tissues in a body. In the present specification, the term "obesity" is to be interpreted in a broad sense, and its concept includes obesity disease. "Obesity disease" is a disease state in need of medically losing weight in the case of having a health problem (complication) due to or relating to obesity, or presumably having such a health problem in the future.
For a determination method of obesity, for example, there is a method of using BMI (body mass index) that is internationally widely used as the criteria of obesity. The BMI is a numerical value obtained by dividing a body weight (kg) by a square of a height (m) (BMI = body weight (kg) /height (m)²). It is determined that BMI < 18.5 is a low weight (underweight), 18.5 ≤ BMI < 25 is a normal weight (normal range), 25 ≤ BMI < 30 is obesity level 1 (preobese), 30 ≤ BMI < 35 is obesity level 2 (obese class I), 35 ≤ BMI < 40 is obesity level 3 (obese class II), and 40 < BMI is obesity level 4 (obese class III) (WHO). In addition, there is a determination method in which a standard body weight (ideal body weight) of a Japanese adult is calculated from the following formula: standard body weight (kg) = height (m)² × 22, by utilizing BMI, and a state that a measured body weight exceeds 120% of the standard body weight (calculated value) is determined as obesity. As a matter of course, a standard body weight (ideal body weight) differs in each individual depending on the sex, age, differences in lifestyle, or the like, and therefore, it is considered not to be appropriate that determination of obesity is uniformly performed by this method.

Preparation of the medicament of the present invention can be carried out in accordance with a conventional method. Upon preparation, other ingredients that are acceptable for preparation (for example, carrier, vehicle, disintegrating agent, buffer, emulsifier, suspending agent, soothing agent, stabilizer, preserving agent, antiseptic agent, physiological saline, etc.) can be contained. As a vehicle, lactose, starch, sorbitol, D-mannitol, white sugar, and the like can be used. As a disintegrating agent, starch, carboxymethyl cellulose, calcium carbonate, and the like can be used. As a buffer, phosphate, citrate, acetate, and the like can be used. As an emulsifier, gum Arabic, sodium arginate, tragacanth, and the like can be used. As a suspending agent, glycerin monostearate, aluminum monostearate, methyl cellulose, carboxymethyl cellulose, hydroxymethyl cellulose, sodium lauryl sulfate, and the like can be used. As a soothing agent, benzyl alcohol, chlorobutanol, sorbitol, and the like can be used. As a stabilizer, propyleneglycol, diethylin sulfite salt, ascorbic acid, and the like can be used. As a preserving agent, phenol, benzalkonium chloride, benzyl alcohol, chlorobutanol, methyl paraben, and the like can be used. As an antiseptic agent, benzalkonium chloride, paraoxybenzoic acid, chlorobutanol, and the like can be used.

A dosage form upon preparation is not particularly limited, and the medicament of the present invention can be provided as, for example, a tablet, a powder, a fine granule, a granule, a capsule, a syrup, an injection agent, an external agent, and a suppository.
In the medicament of the present invention, an active ingredient in an amount required for obtaining expected treatment effects (including a prevention effect) (that is, therapeutically effective amount) is contained. An amount of the active ingredient in the medicament of the present invention generally differs depending on a dosage form thereof, and in order to achieve a desired dosage, the amount of the active ingredient is set within the range of about 0.1% by weight to about 95% by weight, for example.

The medicament of the present invention is applied to a subject by oral administration or parenteral administration (such as intravenous, intraarterial, hypodermic, intramuscular or intraperitoneal injection, percutaneous, nasotracheal or transmucosal administration, etc.) according to a dosage form thereof. The "subject" used herein is not particularly limited and includes a human, mammals other than a human (pet animals, livestock, and experimental animals). Specific examples thereof include mice, rats, guinea pigs, hamsters, monkeys, cattle, pigs, goats, sheep, dogs, cats, chickens, and quails. In a preferable embodiment, the medicament of the present invention is applied to a human.

A dosage of the medicament of the present invention is set in order to obtain expected treatment effects. For setting a therapeutically effective dosage, the symptom, age, sex, and body weight of a patient, and the like are generally considered. It is possible for a person skilled in the art to set an appropriate dosage by considering these matters. For example, the dosage can be set so that an amount of an active ingredient is about 0.1 mg to about 100 mg, preferably about 1 mg to about 50 mg per day, for an adult (body weight of about 60 kg) as a subject. As an administration schedule, for example, one to several doses per day, one dose per two days, or one dose per three days can be adopted. In planning the administration schedule, a medical condition of a patient, an effect retention time of an active ingredient, and the like can be considered.

### Examples

### <Anti-obesity action of GABA_{B} receptor agonist, baclofen>

A study as to whether a GABA_{B} receptor agonist has an anti-obesity action or not was made by using animal models. (±)-baclofen (catalog No. B5399, CAS Number 1134-47-0, Sigma Co.) that is a racemic mixture of baclofen was used as the GABA_{B} receptor agonist. db/db mice that are leptin receptor deficient models were used as the animal models. The db/db mice have obesity, overeating and diabetes, and are frequently used as obese model animals.
In accordance with the method specifically described below, (±)-baclofen was orally administered to the db/db mice for 5 weeks, to examine food intake amounts and body weight changes over time. Further, after administering (±)-baclofen for 5 weeks, examination was performed for a fasting blood glucose level, a blood insulin concentration, a blood leptin concentration, a blood adiponectin concentration, an HbA1c value, a UCP1 mRNA expression amount in brown adipose tissues, a weight of epididymal white adipose tissues and a size of fat cells, a weight of subcutaneous white adipose tissues, and an expression amount of NPY and POMC in the hypothalamic arcuate nucleus.

### 1. Experimental Method

### (1) Method of animal experiment

5 week-old male C57BL/KsJ-db/db mice (db/db mice: body weight 19 to 25 g, Japan SLC, Inc.) were purchased and individually fed in tip cages. For the db/db mice, normal pellet diet (CE-2) was administered under free water intake and free food intake. A body weight and a food intake amount over 24 hours were measured every day from the start of feeding. On day 3 after the start of feeding, blood was taken from an orbit after 6 hour-fasting and a blood glucose level and a blood insulin concentration were measured. The db/db mice were divided into three groups (10 mice per each group) so that a body weight, a 24 hour-food intake amount, a blood glucose level, and a blood insulin concentration were equal, and defined to be a control group, a low concentration baclofen treated group, and a high concentration baclofen treated group. After division into the groups, from day 5 after the start of feeding, (±)-baclofen was dissolved in drinking water and 5 week-continuous oral administration to the baclofen treated groups was started. (±)-baclofen was dissolved in drinking water at a concentration of 1.1 mg/dl in the low concentration treated group and at a concentration of 2.7 mg/dl in the high concentration treated group. A food intake amount and a body weight were measured over time during the period of 5 week-administration of (±) -baclofen, blood was taken after the end of the 5-week administration, and the db/db mice were sacrificed by cervical luxation. Brains, epididymal white adipose tissues, subcutaneous white adipose tissues and brown adipose tissues were surgically removed. The brains and brown adipose tissues were frozen in dry ice and liquid nitrogen immediately after the removal and then preserved at -80°C. The epididymal white adipose tissues and subcutaneous white adipose tissues were measured in weights, and then fixed in 4% formaldehyde and preserved at 4°C.

### (2) Measurement method of blood glucose level and HbA1c

The blood glucose level was measured by a glucose analyzer Antsense (HORIBA, Ltd.) promptly after taking blood from an orbit. HbA1c was measured by a glycohemoglobin analyzer HLC-723 GHbV (manufactured by TOSOH Corporation) after preserving blood at -80°C.

### (3) Measurement method of plasma hormone concentration

After taking blood from an orbit by using a hematocrit tube coated with heparin, blood was centrifuged at 3500 rpm at 4°C for 15 minutes, and plasma was preserved at -80°C. The plasma concentrations of insulin, leptin and adiponectin were measured by the ELISA method (levis insulin kit (mouse): Shibayagi Co., Ltd., Morinaga Leptin assay kit: Morinaga Institute of Biological Science, Inc., Mouse/rat adiponectin ELISA kit: OTSUKA Pharmaceutical Co., Ltd.).

### (4) Histological study of adipose tissue

The epididymal white adipose tissues and subcutaneous white adipose tissues were surgically removed after 5 week-administration of (±)-baclofen, weights of adipose tissues and forms (sizes) of fat cells in the treated groups and the control group were examined by comparison. Comparison of the forms of fat cells was made in the following procedure. That is, the epididymal white adipose tissues preserved with 4% formaldehyde were fixed with paraffin, and then a section with a thickness of 8 µm was prepared to perform Hematoxylin-eosin stain (H-E stain). The forms (sizes) of fat cells were examined by using an image analysis system Neurolucida (MicroBrightField Japan, Inc.).

### (5) In situ hybridization

A hypothalamic section with a thickness of 12 µm was prepared from the brain preserved at -80°C by using a cryostat and stuck to a MAS coat slide and then in situ hybridization was performed. For a label of NPY mRNA and POMC mRNA probes, ³⁵S was used. The hypothalamic section was pretreated by using triethanolamine, ethanol and the like, and then 12 hour-hybridization was performed at 55°C. After the hybridization, the hypothalamic section was treated by washing with SSC, RNase, etc. After development to a film, the mRNA expression strength was assayed by using the NIH image.

### (6) Real time PCR

RNA was taken out from the brown adipose tissues preserved at -80°C, and expression of a UCP-1 mRNA was examined in the real time quantitative PT-PCR method by using ABI PRISM (registered trademark) 7700 Sequence Detection System.

### 2. Result and Prospect

### (1) Food intake suppression, body weight increase suppression, blood leptin concentration change, etc.

Administration of (±)-baclofen in db/db mice significantly suppressed food intake amounts from 1 week after the start of the administration and the significant suppression continued until the end of the experiment (Fig. 1a and Fig. 2a). Further, significant body weight increase suppression was observed in the (±)-baclofen treated groups from 1 week after the start of the administration, and the significant suppression continued until the end of the experiment (Fig. 1d and Fig. 2b).
After administration of (±)-baclofen for 5 weeks, as fasting blood glucose levels (fasting for 6 hours) were compared, significant suppression was observed in the (±)-baclofen treated groups (Fig. 1i). No change was observed in the fasting blood insulin levels (after 5 week-administration) (Fig. 1j). Significant increases in the blood leptin concentrations (after 5 week-administration) were observed in the (±)-baclofen treated groups (Fig. 1m). Significant increases in the blood adiponectin concentrations (after 5 week-administration) were also similarly recognized in the (±)-baclofen treated groups (Fig. 11). Significant decreases in the HbA1c values (after administration for 5 weeks) were observed in the (±)-baclofen treated groups (Fig. 1k). Significant increases in UCP1 mRNA expression in brown adipose tissues (after 5 week-administration) were recognized in the (±)-baclofen treated groups (Fig. 1h). In addition, regarding any of study items, no substantial difference was recognized in the low concentration (±)-baclofen treated group and the high concentration (±)-baclofen treated group.
As described above, in the (±)-baclofen treated groups, significant decreases in the body weight, the food intake amount and the fasting blood glucose level were recognized, and the blood leptin concentration and the blood adiponectin concentration were significantly increased.

### (2) Histological study

Significant decreases in a weight of an epididymal white adipose tissues were recognized in the (±)-baclofen treated groups (Fig. 3a). On the other hand, no significant decrease in a weight of a subcutaneous white adipose tissue was recognized in the (±)-baclofen treated groups (Fig. 3b). Regarding morphological change, it was observed that fat cells became small in the (±) -baclofen treated groups (Fig. 3c, right) as compared with the control group (Fig. 3c, left). When a size of a fat cell was measured, decreases in the size of a fat cell were recognized in the (±)-baclofen treated groups (Fig. 3d). In addition, regarding any of study items, no substantial difference was recognized in the low concentration (±)-baclofen treated group and the high concentration (±)-baclofen treated group.
As described above, it was shown that due to administration of (±)-baclofen, the weight of epididymal white adipose tissues decreased and a size of a fat cell also decreased. That is, it was revealed that the action of (±)-baclofen led to a decrease in a visceral fat level.

### (3) Study of action mechanism

A brain was surgically removed after administration of (±)-baclofen for 5 weeks, and expression of NPY mRNA and POMC mRNA in the hypothalamic arcuate nucleus was examined by using an in situ hybridization method. As a result thereof, expression of NPY mRNA was suppressed due to administration of (±)-baclofen (Fig. 3e, upper left and upper right). Expression of POMC mRNA was enhanced (Fig. 3e, bottom left and bottom right). When quantitative determination of the expression amounts were made by using an NIH image, it was shown that the expression of NPY mRNA was significantly suppressed (Fig. 3f) and the expression of POMC mRNA was significantly enhanced (Fig. 3g) due to administration of (±)-baclofen. In addition, regarding any of study items, no substantial difference was recognized in the low concentration (±) -baclofen treated group and the high concentration (±)-baclofen treated group.
The fact that significant suppression of NPY expression and significant enhancement of POMC expression in the hypothalamic arcuate nucleus were recognized as described above strongly suggests that (±) -baclofen acts on the central nervous system (i.e., via an action on NPY neurons and POMC neurons) and an anti-obesity effect was exerted, which supports effectiveness of baclofen as an anti-obesity medicament. Further, a part of the action mechanism of baclofen was thus revealed and, accordingly, not limited to baclofen, a substance capable of enhancing signaling via a GABA_{B} receptor in the same manner as baclofen, in other words, other GABA_{B} receptor agonists can be prospective candidates for an anti-obesity medicament.

### <Comparison between effects of (R) form baclofen and (S) form baclofen>

Baclofen has been clinically used as a therapeutic agent for spastic paralysis, but a racemic mixture has been used in currently distributed pharmaceutical products. Enantiomers exist for baclofen, and it is reported that, as compared with an S-enantiomer (hereinafter referred to as "S-baclofen"), an R-enantiomer (hereinafter referred to as "R-baclofen") has high binding affinity to a GABA_{B} receptor (Falch, E. et al. Journal of Neurochemistry. 1986. 47: 898-903.). The present inventors performed the following experiment in order to examine differences in a food intake suppression effect and an anti-obesity effect in between R-baclofen and S-baclofen.

### 1. Method of animal experiment

5 week-old male C57BL/KsJ-db/db mice (db/db mice: body weight 19 to 25 g, Japan SLC, Inc.) were purchased and individually fed in tip cages. For the db/db mice, normal pellet diet (CE-2) was administered under free water intake and free food intake. A body weight and a food intake amount over 24 hours were measured every day from the start of feeding. On day 3 after the start of feeding, blood was taken from an orbit after 6 hour-fasting and a blood glucose level and a blood insulin concentration were measured. The db/db mice were divided into three groups (10 mice per each group) so that a body weight, a 24 hour-food intake amount, a blood glucose level, and a blood insulin concentration were equal, and defined to be a control group, an R-baclofen treated group, and an S-baclofen treated group. As R-baclofen, R(+)-Baclofen hydrochloride (catalog No. G013, CAS Number 63701-55-3, Sigma Co.) was used, and as S-baclofen, S(-)-Baclofen hydrochloride (catalog No. G014, CAS Number 63701-56-4, Sigma Co.) was used. After division into the groups, from day 5 after the start of feeding, R-baclofen and S-baclofen were dissolved in drinking water and 5 week-continuous oral administration was started. Both of R-baclofen and S-baclofen were dissolved in drinking water at a concentration of 1.1 mg/dl used in the low concentration treated group in the experiment of baclofen racemic mixture administration. A food intake amount and a body weight were measured over time, and a rectal temperature was measured 5 weeks after the start of the administration. Blood was taken after the 5-week administration of baclofen, and the db/db mice were sacrificed by cervical luxation.

### 2. Result and prospect of experiment

Administration of R-baclofen in db/db mice significantly suppressed a food intake amount and a body weight increase from 2 weeks after the start of the administration, and the significant suppression of both amounts continued until the end of the experiment (Figs. 1b and 1e). On the other hand, in S-baclofen administration, significant suppression of a food intake amount and a body weight increase was not recognized until 4 weeks after the start of the administration, however, significant suppression of both amounts was recognized 5 weeks after the start of the administration (Figs. 1b and 1e). The fact that administration of R-baclofen having high binding affinity to a GABA_{B} receptor had strong effects suggested that baclofen specifically acted on the GABA_{B} receptor and thereby exerted a food intake suppression effect and an anti-obesity effect.

### <Comparison of effects of baclofen in obese model animals (db/db mice) and non-obese model animals (db/⁺m mice)>

A C57BL/KsJ-db/db mouse (db/db mouse) is an obese model animal which develops obesity, overeating and diabetes, but a C57BL/KsJ-db/⁺m mouse (db/⁺m mouse) that is a hetero form of a db/db mouse is a non-obese model animal which does not develop obesity, overeating and diabetes. The present inventors performed the following experiment to examine whether the food intake suppression effect and the body weight increase suppression effect recognized in a db/db mouse by baclofen administration are also recognized in a db/⁺m mouse that is a non-obese model animal or not.

### 1. Method of animal experiment

5 week-old male C57BL/KsJ-db/⁺m mice (db/⁺m mice: body weight 19 to 23 g, Japan SLC, Inc.) were purchased and individually fed in tip cages. To the db/⁺m mice, normal pellet diet (CE-2) was administered under free water intake and free food intake. A body weight and a food intake amount over 24 hours were measured every day from the start of feeding. On day 3 after the start of feeding, blood was taken from an orbit after 6 hour-fasting and a blood glucose level and a blood insulin concentration were measured. The db/⁺m mice were divided into two groups (10 mice per each group) so that a body weight, a 24 hour-food intake amount, a blood glucose level, and a blood insulin concentration were equal, and defined to be a control group and a baclofen treated group. After division into the groups, from day 5 after the start of feeding, (±)-baclofen was dissolved in drinking water and 5 week-continuous oral administration was started in the baclofen treated group. (±)-baclofen was dissolved in drinking water at a concentration of 2.7 mg/dl used in the high concentration treated group in the experiment of db/db mice. A food intake amount and a body weight of the db/⁺m mice were measured over time, and a rectal temperature was measured 5 weeks after the start of the administration. Blood was taken after the 5-week administration of (±)-baclofen, and the db/⁺m mice were sacrificed by cervical luxation.

### 2. Result and prospect of experiment

Administration of (±)-baclofen for 5 weeks in db/⁺m mice did not give significant effects on a food intake amount and a body weight (Figs. 1c and 1f). This fact suggested that a food intake suppression effect and a body weight increase suppression effect due to baclofen might be only recognized in states of overeating and obesity.

### <Influence of baclofen on heat production and comparison of effects thereof in between obese model animal (db/db mouse) and non-obese model animal (db/⁺m mouse)>

A db/db mouse has a low body temperature since heat production thereof is lowered (Trayhurn, P. Pflugers Archiv European Journal of Physiology. 1979. 380: 227-232.), and it is also reported that a body temperature further decreases under diet control since a sufficient food intake amount is required for maintaining the body temperature (Nakagawa, T. et al. Diabetes. 2000. 49: 436-444.). We studied on effects of baclofen administration on a body temperature in db/db mice and db/⁺m mice.

### 1. Method of animal experiment

R-baclofen and S-baclofen were administered to db/db/mice and (±)-baclofen was administered to db/⁺m mice respectively for 5 weeks, and rectal temperatures were then continuously measured for 3 days at 20 o'clock immediately before the dark period when feed intake was started in the mice. The method of measuring rectal temperatures was as follows. That is, the rectal temperatures were measured by using a digital thermistor (manufactured by NATSUME SEISAKUSHO CO., LTD.).

### 2. Result and prospect of experiment

Administration of R-baclofen and S-baclofen significantly increased rectal temperatures in the db/db mice, however, administration of (±)-baclofen did not give a significant effect on rectal temperatures in the db/⁺mmice (Fig. 1g). It can be considered that the fact that administration of baclofen significantly increased body temperatures in the db/db mice to improve low body temperature suggested that baclofen increased heat production in the db/db mice in addition to enhancement of UCP1 mRNA expression in brown adipose tissues, and suggested a possibility that an anti-obesity action of baclofen led to an increase in heat production not only to food intake suppression.

### <Anti-obesity action of baclofen in other obese model animals>

A db/db mouse is an obese model animal which develops obesity and diabetes accompanied by deficiency of a leptin receptor, and a diet-induced obese mouse (DIO mouse) was prepared by administering high fat-diet to a normal mouse as a typical example of an obese model animal closer to human obesity disease, and an anti-obesity action of baclofen was examined. In addition, an action in a non-obese mouse (lean mouse) prepared by administering a normal diet to a normal mouse of the same species was also examined in comparison.

### 1. Experimental method

5 week-old male C57BL/6N mice (body weight 17 to 20 g, CLEA Japan, Inc.) were purchased and individually fed in tip cages. To the mice, normal pellet diet (CE-2) was administered under free water intake and free food intake. A body weight and a food intake amount over 24 hours were measured every day from the start of feeding. On day 3 after the start of feeding, blood was taken from an orbit after 6 hour-fasting and a blood glucose level and a blood insulin concentration were measured. The mice were divided into a control group and a baclofen treated group (10 mice per each group) so that a body weight, a 24 hour-food intake amount, a blood glucose level, and a blood insulin concentration were equal, and diet induced-obesity (DIO) was started to be induced by administration of high fat diet (High Fat Diet 32, energy fat ratio 60%, CLEA Japan, Inc.) (DIO mice). At the same time as the start of administering the high fat diet, (±)-baclofen was dissolved in drinking water and 5 week-continuous oral administration was started in a dosage of 5 mg per 1 kg of a body weight in 1 day (5 mg/kg/day). A food intake amount and a body weight were measured over time during the 5 week-administration period. (±)-baclofen (5 mg/kg/day) was orally administered also to C57BL/6N mice (lean mice) that are non-obese mice to which normal diet was administered in the same manner as described above, and a food intake amount and a body weight were measured over time.

### 2. Result and prospect of experiment

Administration of (±)-baclofen to the DIO mice significantly suppressed a food intake amount and a body weight increase from 1 week after the start of the administration, and significant suppression of both amounts continued until after 5 weeks when the experiment was ended (Figs. 4a and 4b, Figs. 5a and 5b). On the other hand, administration of (±)-baclofen in the lean mice did not give significant effects on a food intake amount and a body weight during the administration period (Figs. 4c and 4d). The above results suggested that an anti-obesity action of baclofen was also recognized in DIO mice that were obese model animals closer to human obesity disease and an effect of suppression of a body weight increase was only recognized in an obese state.

### <Anti-obesity action of GABA_{B} receptor agonist other than baclofen>

For the purpose of examining whether administration of other GABA_{B} receptor agonists which differ in structural formulae from baclofen gives an anti-obesity action in an obese model animal in the same manner as baclofen or not, 3-aminopropyl-(methyl) phosphinic acid (catalog No. EA-133, CAS Number: 127729-35-5. BIOMOL Co.) (hereinafter referred to as SKF97541) and 3-aminopropylphosphonic acid (catalog No. 268615, CAS Number: 13138-33-5, Sigma-Aldrich Corporation) (hereinafter referred to as 3-APPA) were continuously orally administered to DIO mice to examine anti-obesity actions thereof. Further, actions in non-obese mice (lean mice) prepared by administering normal diet to normal mice of the same species were also examined in comparison. Structural formulae of SKF97541 and 3-APPA are shown in Fig. 13.

### 1. Experimental method

5 week-old male C57BL/6N mice (body weight 17 to 20 g, CLEA Japan, Inc.) were purchased and individually fed in tip cages. Normal pellet diet (CE-2) was administered to the mice under free water intake and free food intake. A body weight and a food intake amount over 24 hours were measured every day from the start of feeding. On day 3 after the start of feeding, blood was taken from an orbit after 6 hour-fasting and a blood glucose level and a blood insulin concentration were measured. The mice were divided into a control group, an SKF97541 treated group and a 3-APPA treated group (10 mice per each group) so that a body weight, a 24 hour-food intake amount, a blood glucose level, and a blood insulin concentration were equal, and diet induced-obesity (DIO) was started to be induced by administration of high fat diet (High Fat Diet 32, energy fat ratio 60%, CLEA Japan, Inc.) (DIO mice). At the same time as the start of administering the high fat diet, SKF97541 (0.3 mg/kg/day) or 3-APPA (5 mg/kg/day) was dissolved in drinking water and 5 week-continuous oral administration was started. A food intake amount and a body weight were measured over time during the 5 week-administration period. SKF97541 (0.3 mg/kg/day) or 3-APPA (5 mg/kg/day) was orally administered also to C57BL/6N mice (lean mice) to which normal diet was administered in the same manner as described above, and a food intake amount and a body weight were measured over time.

### 2. Result and prospect of experiment

Administration of SKF97541 or 3-APPA to the DIO mice significantly suppressed a food intake amount and a body weight increase from 1 week after the start of the administration, and significant suppression of both amounts continued until after 5 weeks when the experiment was ended (Figs. 4e and 4f, Figs. 5a and 5b). On the other hand, administration of SKF97541 or 3-APPA in the lean mice did not give significant effects on a food intake amount and a body weight during the administration period (Figs. 4g and 4h). The above results suggested that an anti-obesity action was recognized in DIO mice that were obese model animals by the three different kinds of GABA_{B} receptor agonists and an effect of suppressing a body weight due to these GABA_{B} receptor agonists was only recognized in an obese state.

### <Effects of GABA_{B} receptor agonist on glucose tolerance and insulin sensitivity in DIO mice>

Examination was performed for changes in fasting blood glucose levels, HbA1c values and fasting blood insulin concentrations when three kinds of GABA_{B} receptor agonists were continuously administered to DIO mice.

### 1. Experimental method

(±)-baclofen (5 mg/kg/day), SKF97541 (0.3 mg/kg/day) or 3-APPA (5 mg/kg/day) was orally administered to DIO mice for 5 weeks according to the above described method, and then blood was taken from an orbit after 6 hour-fasting and a blood glucose level, an HbA1c value and a blood insulin concentration were measured.

### 2. Result and prospect of experiment

In any of the (±)-baclofen treated group, the SKF97541 treated group, and the 3-APPA treated group, significant decreases in fasting blood glucose levels (Fig. 6), HbA1c values (Fig. 7) and fasting blood insulin concentrations (Fig. 8) were recognized as compared with the control group. The above results suggested that glucose tolerance and insulin sensitivity in DIO mice were improved by the three different kinds of GABA_{B} receptor agonists.

### <Other effects of (±)-baclofen in DIO mouse>

Examination was performed for change in a plasma hormone concentration and an effect on white adipose tissues when (±)-baclofen was continuously administered to DIO mice.

### 1. Experimental method

(±)-baclofen (5 mg/kg/day) was orally administered to DIO mice for 5 weeks according to the above described method, and then, blood was taken from an orbit after 6 hour-fasting, and a plasma leptin concentration and a plasma adiponectin concentration were measured. The DIO mice were sacrificed by cervical luxation after the end of the 5-week administration, and then epididymal white adipose tissues and subcutaneous white adipose tissues were surgically removed, and after measuring the weights, they were fixed in 4% formaldehyde. The epididymal white adipose tissues were cut out to prepare a section with a thickness of 8 µm and subjected to Hematoxylin-Eosin stain (H-E stain) and a form (size) of a fat cell was examined by using an image analysis system, Neurolucida (MicroBrightField Japan, Inc.).

### 2. Result and prospect of experiment

The 5 week-oral administration of (±)-baclofen in the DIO mice significantly decreased a plasma leptin concentration (Fig. 9a), and significantly increased a plasma adiponectin concentration (Fig. 9b). Further, the administration significantly decreased the weight of epididymal white adipose tissues (Fig. 10a) and a weight of subcutaneous white adipose tissues (Fig. 10b). A size of a fat cell in the epididymal white adipose tissues was decreased in the (±) -baclofen treated group (Fig. 10c). It was suggested that a white adipose weight and a size of a fat cell decreased due to (±)-baclofen administration.

### <Action mechanism of (±)-baclofen in DIO mice>

Examination was performed for changes in expression amounts of NPY mRNA and POMC mRNA in the hypothalamic arcuate nucleus when (±) -baclofen was continuously administered to the DIO mice.

### 1. Experimental method

(±)-baclofen was orally administered to DIO mice for 5 weeks according to the above method, and then the DIO mice were sacrificed. A brain was surgically removed, and changes in expression amounts of NPY mRNA and POMC mRNA in the hypothalamic arcuate nucleus were examined by using an in situ hybridization method.

### 2. Result and prospect of experiment

The administration of (+)-baclofen to the DIO mice significantly suppressed expression of NPY mRNA (Figs. 11a and 11c), and significantly increased expression of POMC mRNA (Figs. 11b and 11c). A possibility that (±)-baclofen improves obesity disease by acting on the appetite center in DIO mice similarly to db/db mice was suggested.

### <Effects of baclofen on body temperature, oxygen consumption, and motor activity>

Effects on body temperatures, oxygen consumptions, and motor activity in db/db mice and DIO mice by (±)-baclofen administration were examined.

### 1. Experimental method

(±)-baclofen was orally administered to db/db mice and DIO mice for 5 weeks and rectal temperatures were measured 5 weeks after the start of the administration, and oxygen consumptions and motor activity were measured by using a small animal metabolism measuring system (model MK-5000: Muromachi Kikai Co., Ltd.).

### 2. Result and prospect of experiment

Administration of (±)-baclofen to db/db mice and DIO mice significantly raised body temperatures (Figs. 12a and 12d), and significantly increased oxygen consumptions (Figs. 12b and 12e). The administration did not give a significant effect on motor activity (Figs. 12c and 12f). The above results suggested a possibility that at least a part of an anti-obesity action by baclofen was via enhancement of energy production.

### <Study of dosage dependency in anti-obesity action of baclofen, GABA_{B} receptor agonist>

The following experiment was performed for the purpose of clarifying dosage dependency of an anti-obesity action of baclofen that is a GABA_{B} receptor agonist. (±)-baclofen (catalog No. B5399, CAS Number 1134-47-0, Sigma Co.), which is a racemic mixture, was used as baclofen. db/db mice that are leptin receptor deficient models were used as the experimental animals.

### 1. Experimental method

5 week-old male C57BL/KsJ-db/db mice (db/db mice: body weight 19 to 25 g, Japan SLC, Inc.) were purchased and individually fed in tip cages. Normal pellet diet (CE-2) was administered to the db/db mice under free water intake and free food intake. A body weight and a food intake amount over 24 hours were measured every day from the start of feeding. On day 3 after the start of feeding, blood was taken from an orbit after 6 hour-fasting, and a blood glucose level and a blood insulin concentration were measured. The db/db mice were divided into five groups (10 mice per each group) so that a body weight, a 24 hour-food intake amount, a blood glucose level, and a blood insulin concentration were equal, and defined to be a control group and baclofen treated groups. (±)-baclofen was dissolved in drinking water at respective concentrations of 4 x 10⁻⁶ M, 10 × 10⁻⁶ M, 40 × 10⁻⁶ M, and 100 × 10⁻⁶ M, and continuously orally administered to the baclofen treated groups for 5 weeks. A food intake amount, a body weight and a water-drinking amount were measured over time during the period of 5 week-administration of (±)-baclofen.

### 2. Result and prospect of experiment

The administration of (±)-baclofen to db/db mice significantly suppressed a food intake amount and a body weight increase from 1 week after the start of the administration in the 100 × 10⁻⁶ M and 40 × 10⁻⁶ M treated groups, and significant suppression of both amounts continued until the end of the experiment. Significant suppression of a food intake amount and a body weight increase was recognized from 2 weeks after the start of the administration in the 10 × 10⁻⁶ M treated group and from 4 weeks after the start of the administration in the 4 × 10⁻⁶ M treated group, and significant suppression of the both amounts continued until the end of the experiment (Figs. 14a and 14b). Roughly calculated dosages of baclofen per day were 5 mg/kg/day for 100 × 10⁻⁶ M, 2 mg/kg/day for 40 × 10⁻⁶ M, 0.5 mg/kg/day for 10 × 10⁻⁶ M, and 0.2 mg/kg/day for 4 × 10⁻⁶ M.

### <Study of anti-obesity action of GABA_{B} receptor agonists other than baclofen in db/db mouse>

For the purpose of examining whether administration of GABA_{B} receptor agonists other than baclofen gives an anti-obesity action in a db/db mouse or not, 3-aminopropyl-(methyl) phosphinic acid (catalog No. EA-133, CAS Number: 127729-35-5, BIOMOL Co.) (hereinafter referred to as SKF97541) and 3-aminopropylphosphonic acid (catalog No. 268615, CAS Number: 13138-33-5, Sigma-Aldrich Corporation) (hereinafter referred to as 3-APPA) were continuously orally administered to db/db mice to examine anti-obesity actions thereof. Further, actions in db/⁺m mice that are non-obese model animals were also examined in comparison.

### 1. Experimental method

5 week-old male C57BL/KsJ-db/db mice (db/db mice: body weight 19 to 25 g, Japan SLC, Inc.) were purchased and individually fed in tip cages. Normal pellet diet (CE-2) was administered to the db/db mice under free water intake and free food intake. A body weight and a food intake amount over 24 hours were measured every day from the start of feeding. On day 3 after the start of feeding, blood was taken from an orbit after 6 hour-fasting and a blood glucose level and a blood insulin concentration were measured. The db/db mice were divided into three groups (10 mice per each group) so that a body weight, a 24 hour-food intake amount, a blood glucose level, and a blood insulin concentration were equal and defined to be a control group, an SKF97541 treated group and a 3-APPA treated group. SKF97541 and 3-APPA were dissolved in drinking water at respective concentrations of 10 × 10⁻⁶ M and 100 × 10⁻⁶ M, and continuously orally administered for 5 weeks, and a food intake amount and a body weight were measured over time during the administration period. SKF97541 (10 × 10⁻⁶ M) or 3-APPA (100 × 10⁻⁶ M) was dissolved in drinking water and orally administered also to db/⁺m mice, and a food intake amount and a body weight were measured over time in the same manner.

### 2. Result and prospect of experiment

Administration of 3-APPA and SKF97541 significantly suppressed cumulative food intake amounts (Fig. 15a) respectively from 1 week after the start of the administration and from 2 weeks after the start of the administration, and the significant suppression continued until the end of the experiment. Further, the administration of SKF97541 and 3-APPA significantly suppressed body weight increases (Fig. 15b) from 1 week after the start of the administration and the significant suppression continued until the end of the experiment. On the other hand, the administration of SKF97541 and 3-APPA to db/⁺m mice did not give significant effects on a food intake amount and a body weight during the administration period (Figs. 15c and 15d). The above results suggested that an anti-obesity action was recognized in db/db mice due to the three different kinds of GABA_{B} receptor agonists and the anti-obesity action was recognized only in an obese state.

### Industrial Applicability

The anti-obesity agent of the present invention is advantageous for treatment or prevention of obesity (including obesity disease). With to the anti-obesity agent of the present invention, a preferable anti-obesity effect can be obtained by a central action of due to an active ingredient thereof.

The present invention is not limited to the above descriptions of embodiments and examples of the present invention. Various modifications are included in the invention within the range that a skilled person in the art can easily conceive without departing from the description of the scope of claims.

## Claims

1. A medicament comprising a GABA_{B} receptor agonist or a pharmacologically acceptable salt thereof as an active ingredient for use in the treatment or prevention of obesity, wherein the medicament is designed to be applied to a human and the dosage of the amount of the active ingredient is 0.1 mg to 100 mg per day.

2. The medicament of claim 1, wherein the dosage of the amount of the active ingredient is 1 mg to 50 mg per day.

3. The medicament according to claim 1 or 2, wherein said GABA_{B} receptor agonist is baclofen.

4. The medicament according to claim 3, wherein said baclofen is in the (R) form.

5. The medicament according to any one of claims 1 to 4, wherein the medicament acts on NPY neurons and POMC neurons in the hypothalamic arcuate nucleus.

6. The medicament according to any one of claims 1 to 5, wherein obesity is due to leptin resistance or accompanied by leptin resistance.

7. The medicament according to any one of claims 1 to 6, which is designed to be applied by oral administration.

## Patentansprüche

1. Medikament umfassend einen GABA_{B} Rezeptor-Agonisten oder ein pharmakologisch verträgliches Salz daraus als einen aktiven Bestandteil zur Verwendung in der Behandlung oder Prävention von Obesitas, wobei das Medikament zum Gebrauch im Menschen hergerichtet ist und die Dosierung 0,1 mg bis 100 mg pro Tag der Menge an aktiven Bestandteil beträgt.

2. Medikament nach Anspruch 1, wobei die Dosierung 1 mg bis 50 mg pro Tag der Menge an aktiven Bestandteil ist.

3. Medikament nach einem der Ansprüche 1 oder 2, wobei der GABA_{B} Rezeptor-Agonist Baclofen ist.

4. Medikament nach Anspruch 3, wobei das Baclofen in der (R) Form vorliegt.

5. Medikament nach einem der Ansprüche 1 bis 4, wobei das Medikament auf NPY Neuronen und POMC Neuronen im hypothalamischen Nucleus arcuatus agiert.

6. Medikament nach einem der Ansprüche 1 bis 5, wobei Obesitas aufgrund einer Leptinresistenz oder eine Leptinresistenz begleitend auftritt.

7. Medikament nach einem der Ansprüche 1 bis 6, welches für eine orale Verabreichung hergerichtet wird.

## Revendications

1. Médicament comprenant un agoniste du récepteur GABA_{B} ou un sel pharmaceutiquement acceptable de celui-ci à titre d'ingrédient actif pour l'utilisation dans le traitement ou la prévention de l'obésité, le médicament étant destiné à être administré à un humain et le dosage de la quantité d'ingrédient actif étant de 0,1 mg à 100 mg par jour.

2. Médicament selon la revendication 1, dans lequel le dosage de la quantité d'ingrédient actif est de 1 mg à 50 mg par jour.

3. Médicament selon la revendication 1 ou 2, dans lequel ledit agoniste du récepteur GABA_{B} est le baclofène.

4. Médicament selon la revendication 3, dans lequel ledit baclofène étant sous forme (R).

5. Médicament selon l'une quelconque des revendications 1 à 4, dans lequel le médicament agit sur les neurones NPY et les neurones POMC dans le noyau arqué hypothalamique.

6. Médicament selon l'une quelconque des revendications 1 à 5, dans lequel l'obésité est due à la résistance à la leptine ou est accompagnée par une résistance à la leptine.

7. Médicament selon l'une quelconque des revendications 1 à 6, dans lequel qui est destiné à être administré par voie orale.
